# EUROPEAN PATENT SPECIFICATION

(11) **EP 1 896 598 B1**
(45) Date of publication and mention of the grant of the patent: **05.11.2008**
(21) Application number: 06777512.2
(22) Date of filing: 29.06.2006
(51) Int. Cl.: C12P 7/28, C12P 19/26, C12P 17/12

(54) **ENZYMATIC METHOD FOR PRODUCING DIHYDROXYACETONE PHOSPHATE**
ENZYMATISCHES VERFAHREN ZUR HERSTELLUNG VON DIHYDROXYACETONPHOSPHAT
PROCEDE ENZYMATIQUE DE PRODUCTION DE LA DIHYDROXYACETONE PHOSPHATE

(30) Priority: 30.06.2005 EP 05105913; 01.07.2005 EP 05106005
(43) Date of publication of application: 12.03.2008
(73) Proprietor: Universiteit van Amsterdam, 1012 WX Amsterdam (NL)
(72) Inventor: VAN HERK, Teunie, NL-1113 HB Diemen (NL); HARTOG, Aloysius, Franciscus, NL-2015 AG Haarlem (NL); WEVER, Ronald, NL-1622 HG Hoorn (NL)
(74) Representative: Metman, Karel Johannes
(86) International application number: PCT/EP2006/063686
(87) International publication number: WO 2007/003574

(56) References cited:
- SCHOEVAART R ET AL: "A four-step enzymatic cascade for the one-pot synthesis of non-natural carbohydrates from glycerol." THE JOURNAL OF ORGANIC CHEMISTRY. 20 OCT 2000, vol. 65, no. 21, 20 October 2000 (2000-10-20), pages 6940-6943, XP002354659 ISSN: 0022-3263 cited in the application
- SIMON E S ET AL: "PREPARATION OF PHOSPHOENOLPYRUVATE FROM D-LEVO-3 PHOSPHOGLYCERIC ACID FOR USE IN REGENERATION OF ATP" JOURNAL OF THE AMERICAN CHEMICAL SOCIETY, vol. 111, no. 24, 1989, pages 8920-8921, XP002354660 ISSN: 0002-7863 cited in the application
- TANAKA NAOKO ET AL: "Phosphorylation and dephosphorylation of polyhydroxy compounds by class A bacterial acid phosphatases." ORGANIC & BIOMOLECULAR CHEMISTRY. 21 AUG 2003, vol. 1, no. 16, 21 August 2003 (2003-08-21), pages 2833-2839, XP002354661 ISSN: 1477-0520 cited in the application
- STUKEY JOSEPH ET AL: "Identification of a novel phosphatase sequence motif" PROTEIN SCIENCE, vol. 6, no. 2, 1997, pages 469-472, XP002354662 ISSN: 0961-8368 cited in the application
- SCHOEVAART R ET AL: "Class I fructose-1,6-bisphosphate aldolases as catalysts for asymmetric aldol reactions" TETRAHEDRON: ASYMMETRY, ELSEVIER SCIENCE PUBLISHERS, AMSTERDAM, NL, vol. 10, no. 4, 26 February 1999 (1999-02-26), pages 705-711, XP004222843 ISSN: 0957-4166

## Description

The invention relates to a method for making dihydroxyacetone phosphate and to methods for making stereospecific products.

Dihydroxyacetone phosphate (DHAP) is a versatile substrate in the stereoselective formation of C-C bonds in aldol condensation reactions using DHAP dependent aldolase enzymes. The synthetic utility of the aldolases is very large. Since DHAP-dependent aldolases are very specific for the donor substrate, the availability of DHAP has been an important issue for the development of the practical usage of these enzymes. Although commercially available, DHAP is too expensive for enzymatic synthesis on a large scale; hence, several chemical and enzymatic methods for generating DHAP have been developed. However, most of these methods yield complex substrate mixtures containing phosphorylated substances other than DHAP. These by-products are hard to be separated from DHAP and often have a strong inhibitory effect on aldolases. Among these methods, chemical synthesis of DHAP provides the cleanest product but needs multiple protection and deprotection steps. The use of relatively pure DHAP also favors the formation of the aldol adducts and simplifies the purification process.

Simon et al., J. Am. Chem. Soc., 1989, 111, 8920-8921 have disclosed a synthesis of DHAP from dihydroxyacetone. ATP is used as the phosphate donor to phosphorylate dihydroxyaceton (DHA) to dihydroxyacetone phosphate by glycerol kinase according to Scheme A:

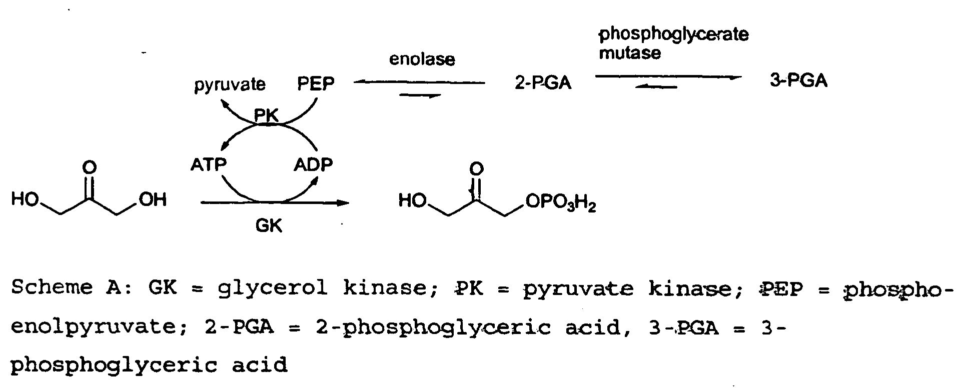

This method, however, has various disadvantages. ATP is very expensive and cannot be used in stoichiometric amounts, for which reason the ADP formed has to be reconverted into ATP by another phosphate donor. Simon has described to use phosphoenolpyruvaat (PEP) and pyruvate kinase as the other donor, which is very expensive and makes this process commercially unattractive. Alternatively, PEP is not purchased but prepared from D-(-)-3-phosphoglyceric acid (3-PGA). This necessitates a complex reaction scheme wherin 3-PGA has to be converted into 2-PGA by a phosphoglycerate mutase, after which 2-PGA is converted into PEP by an enolase. Thus this method needs four different enzymes to convert DHA into DHAP: phosphoglycerate mutase, enolase, pyruvate kinase and glycerol kinase. This is not only extremely complicated but also difficult because the activity of four different enzymes is difficult to control and the pH should be controlled meticulously by using a pH stat. Finally, also 3-PGA is a rather expensive material.

Other disadvantages of this method are that large amounts of pyruvate are produced which interfere with the isolation of DHAP and that these glycolytic enzymes are easily inactivated by oxygen, which needs careful procedures under nitrogen atmosphere.

An improvement of this commercially unattractive procedure was disclosed by Schoevaart et al., J. Org. Chem., 2000, 65, 6940-6943, as illustrated in Scheme B:

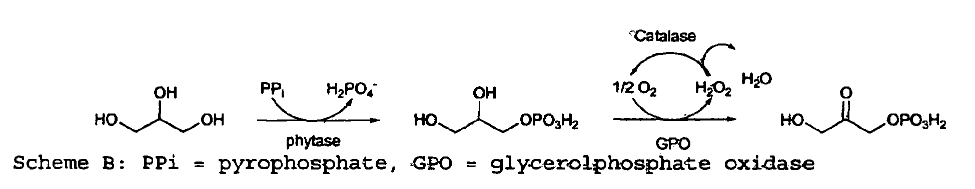

According to this method DHAP is not directly made from dihydroxyacetone but from glycerol which is phosphorylated by phytase to give a racemic mixture of glycerol phosphates. Moreover, this procedure needs an oxidation step and glycerolposphate oxidase is used to oxidize the glycerolphosphate to DHAP. GPO is only able to convert the L-isomer, which means that 50% of the mixture is not converted and thus lost. Further a second enzyme is necessary, i.e. catalase that should be present to prevent inactivation of the glycerolphosphate oxidase by the hydrogen peroxide formed. The process should be performed in a two-step procedure since the pH has to be changed twice and gives an extremely low conversion of 1% of glycerol to the end product.

According to these prior art methods DHAP is too expensive to be commercially used in organic synthesis. It is therefore an objective of the present invention to provide a cheap and simple method for making DHAP, which can be performed as a one-pot procedure, has a high conversion, makes use of one enzyme only, and makes use of commonly available starting materials that can be obtained at low costs in bulk quantities.

To this end the invention pertains to a method for making dihydroxyacetone phosphate (DHAP) comprising treating dihydroxyacetone with a bacterial acid phosphatase in the presence of pyrophosphate. Only one single enzyme is needed: the phosphatase.

DHAP is mandatory for the DHAP dependent aldolase, and the need for DHAP in large quantities requires efficient synthesis to be worked out. A major problem of dihydroxyacetone phosphate is its instability in aqueous environment. This compound is quite labile. This may limit the operating variables for the production of DHAP. If long operating times have to be accepted due to economic reasons in view of high enzyme costs, low pH and/or temperatures lower than 35° C are preferred. In addition most of the common enzymes of interest for the production of DHAP have their pH-optimum in the neutral of the alkaline pH range.

Phosphatases form a class of enzymes that are able to hydrolyze a wide range of phosphorylated compounds resulting in the liberation of phosphate and the corresponding alcohol. In the context of the present invention the term phosphatase is intended to mean an enzyme that is able to transphosphorylate various chemical compounds using pyrophosphate as the phosphate donor resulting in a variety of phosphorylated compounds.

To date phosphatases are widely spread in nature and have been isolated from several sources, e.g. mammals (glucose-6-phosphatase), plants, and in particular from bacterial species

Suitable bacterial species for providing the phosphatase include *Salmonella typhimurium, Salmonella enterica Zymomonas mobilis, Morganella morganii, Shigella flexneri, Escherichia blattae*, *Klebsiella planticola*, *Prevotella intermedia,* and mutants that have improved activity. Preferred enzyme is obtained from *Shigella flexneri*. These enzymes are know to be homologous to each other (Y. Mihara, T. Utagawa, H. Yamada, and Y. Asano, J. Bioscience. Bioeng., 92 (2001) 50-54) and share the three conserved sequence motifs domain 1: KXXXXXXRP, domain 2: PSGH and domaine 3: SRXXXXXHXXXD, which are known and identified by J. Stukey J and G.M. Carman, Protein Sci., 6 (1997) 469-472 and by W. Hemrika, R. Renirie, , H.L. Dekker, B. Barnett, and R. Wever, Proc. Natl. Acad. Sci. USA, 94 (1997) 2145-2149.

In the method enzyme variants that have the above degree of homology to the polypeptide or to recombinant enzymes that are obtained by introduction of a mutation into the nucleic acid sequence coding for the polypeptide and that have a better or modified enzymatic activity and share the three conserved sequence motifs domain 1: KXXXXXXRP, domain 2: PSGH and domain 3: SRXXXXXHXXXD can be used. These mutations may be obtained from the parent gene by site directed mutagenesis or by directed evolution techniques.

This method allows to use cheap pyrophosphate instead of expensive phosphate donors such as ATP or 3-PGA, and cheap starting materials (dihydroxyacetone; DHA), whereas no organic side products are generated during the reaction, and high yields are obtained.

The aldol reaction, which creates a carbon-carbon bond as well as two contiguous stereogenic centers, is of vital interest for organic synthesis. The potential advantages of enzyme-catalyzed aldol reactions, i.e., mild reaction conditions, enantioselectivity, and reduction of waste, have stimulated research in this field. The DHAP dependent aldolases, which in their natural role perform a retro-aldol reaction, have the advantage of nearly absolute control over the newly created stereogenic centers. A complete set of four known DHAP aldolases of complementary specificity with respect to the stereochemistry of the two C-atoms connected by the newly formed C-C bond are known, see for instance Schoevaart et al.. Extensive studies have demonstrated that these enzymes accept a wide range of aldehydes as the acceptor substrates. These enzymes have been used mainly to synthesize a wide variety of mostly unusual carbohydrates which are then potentially useful as chiral building blocks for the synthesis of biologically active compounds.

The invention therefore also has for its object to provide a method for making stereospecifically a phosphate of the formula:

R-C*H (OH) -C*H (OH) -CO-CH₂-OPO₃H₂

which method comprises the steps:
a) treating dihydroxyacetone with a bacterial acid phosphatase in the presence of pyrophosphate to make dihydroxyacetone phosphate; and
b) treating the dihydroxyacetone phosphate with R-CHO and an aldolase, wherein R-CHO is any aldehyde, preferably R is selected from H, unsubstituted or substituted (cyclo)alkyl, and a carbohydrate moiety that may contain fluor, sulfur, or nitrogen, and C* stands for a chiral carbon atom.

Step b) is a known reaction step which is described in the literature. Many aldehydes are described for use in this reaction step, for instance in the reference work of K. Faber, Biotransformations in Organic Chemistry, 4th Ed., Springer Verlag, p. 277-279. Particularly useful aldehydes are R-CHO wherein R is selected from H, unsubstituted or substituted (cyclo) alkyl, and a carbohydrate moiety. The expression "(cyclo) alkyl" means alkyl or cycloalkyl, which may be branched or unbranched. Dependent on the required end product other aldehydes can also be used, for instance having unsaturated hydrocarbon groups, peptides, and the like. Substituents can be groups such as hydroxy, halogen, amino, (di) alkylamino, nitro, keto, carboxylate, carboxamide, CN, azido, sulfur, and the like.

These stereospecific phosphates are usually not the commercially desired end products, and it is therefore also an objective to remove said phosphate group to obtain useful end products. Chemical and enzymatic methods for removing the phosphate group introduced into the product as a part of the DHAP moiety can be used such as acid-catalyzed hydrolysis using soluble acids or cation-exchange resins and hydrolysis catalyzed by acid phosphatase or alkaline phosphatase. The most useful procedure uses acid phosphatases. In general, the nonenzymatic routes cause too much decomposition of the aldol adducts to be useful, although for certain acid-stable products this method can be used. The values of pH required (pH 8-9) for reasonable activity of alkaline phosphatase may also cause decomposition and reactions other than hydrolysis of phosphate in the case of adducts that are sensitive to base. Acid phosphatase operates in a pH range (pH 5-7) tolerated by most adducts. It accepts a wide range of substrates, is inexpensive, is stable, and is easy to manipulate. Acid phosphatase may be used in soluble form, but containment within a dialysis membrane or in immobilized form is more convenient on a large scale because separation of the protein from the reaction mixture is then simplified. An acid phosphatase is already present in the redaction mixture to phosphorylate DHA initially and is still active to dephosphorylate the aldol adduct.

The invention therefore in another embodiment relates to a method for making stereospecifically a compound of the formula:

R- C*H (OH) -C*H (OH) -CO-CH₂ -OH

comprising the steps:
a) treating dihydroxyacetone with a bacterial acid phosphatase in the presence of pyrophosphate to make dihydroxyacetone phosphate;
b) treating the dihydroxyacetone phosphate with R-CHO and an aldolase, wherein R-CHO is any aldehyde, preferably R is selected from H, unsubstituted or substituted (cyclo) alkyl, and a carbohydrate moiety, and C* stands for a chiral carbon atom to a phosphate of the formula:

   R-C*H (OH) -C*H (DH) -CO-CH₂-OPO₃H₂; and
c) dephosphorylating the phosphate of step b) by treating said phosphate with a bacterial acid Phosphatase.

Since most enzymes operate at room temperature in aqueous solution, their reactions are often compatible with each other and can be carried out at a single pH. This makes it possible to combine several enzymes in a one-pot, multistep reaction sequence. Their use in aqueous solution and their biodegradability make enzymes also an excellent .environmentally acceptable option. The high regio- and stereoselectivity and catalytic efficiency make enzymes specially useful for the synthesis of complex, highly functionalized molecules like carbohydrates.

D-Fructose 1,6-bisphosphate aldolase from rabbit muscle (RAMA) is the aldolase most widely employed for preparative synthesis owing to its commercial availability. This class I aldolase, which reversibly catalyzes the formation of D-fructose 1,6-bisphosphate by the reversible addiction of DHAP to D-glyceraldehyde 3-phosphate, has been used extensively because of its broad tolerance for various other aldehydes acceptors. Other aldolases can also be used, for instance, DHAP-dependent aldolase belonging to the class selected from rhamnulose 1-phosphate aldolase, D-tagatose 1,6-bis-phosphate aldolase, and L-fuculose 1-phosphate aldolase.

The invention is illustrated by the following non-limitative examples.

### General

### Formation of DHAP

A reaction mixture was used containing 100 mM DHA, 100 mM PPi in 100 mM acetate buffer pH 5. 5 µM phosphatase (obtained from Shigella flexneri (PhoN-Sf)) yielded a maximum DHAP concentration of 4 mM at 20 minutes incubation time. Lowering the PhoN-Sf concentration increases the DHAP yield, but also lengthens the time to reach this maximum concentration. 1 µM PhoN-Sf gives a concentration of 4.5 mM of DHAP in 110 minutes.

The phosphorylating activity of PhoN-Sf was highly dependent on pH. The highest DHAP concentration was reached with pH 4. A concentration of 5.5 mM DHAP was seen after 100 minutes of incubation (2 µM PhoN-Sf, 100 mM of DHA and PPi) . Increasing the pH increased the initial velocity of DHAP formation, but also the hydrolyses of DHAP was fastened. At pH 6, only 2 mM of DHAP was formed. At pH the transphosphorylation reaction was not finished within this time scale. Transphosphorylation is very slow, but also, the hydrolysis of PPi and the formed DHAP was slower. The same pH dependency has been observed in the phosphorylation of inosine to 5'-inosine monophosphate by PhoN-Sf.

The phosphorylating activity is also dependant on the DHA concentration. The yield increases from 5 mM to 67 mM DHAP from 100 mM to 2.5 M DHA, using 100 mM PPi and 2 µM PhoN-Sf. The Km value for DHA is quite high, over 1 M. Maximum concentration in a 100 mM DHA reaction mixture was reached at 50 minutes. Increasing the DHA concentration caused the maximum concentration to be reached earlier.

When using 100 mM PPi 5 mM DHAP was formed using 100 mM DHA and 2 µM PhoN-Sf. Doubling the PPi concentration to 200 mM gave 8 mM of DHAP.

Combining optimization factors increases the DHAP concentration considerably. A reaction mixture containing 500 mM DHA and 240 mM PPi at pH 4.5 was combined with 2 µM PhoN-Sf. 52 mM of DHAP was formed. Addition of a second portion of 240 mM PPi after 100 minutes pushed the yield to 104 mM of DHAP. The Km value of DHAP for RAMA is 50 µM resulting in an effective aldol condensation with the aldehyde and preventing the dephosphorylation of DHAP, thereby increasing the efficiency of the overall reaction.

Expression and purification of recombinant Shigella flexneri PhoN (PhoN-Sf) was as described elsewhere [N. Tanaka, Org. Biomol . Chem., 1, 2833-2839)]. All chemicals and other enzymes were purchases from commercial suppliers and used without purification.

### Enzymatic DHAP assay

DHAP was assayed with a coupled enzyme system: reduction with NADH-consuming glycerol-3-phosphate dehydrogenase enables determination of DHAP by measuring NADH concentration with UV spectroscopy. From a diluted DHAP solution, 40 µl was added in a quartz cuvette containing 1.96 mL of 50 mM TRIS pH 7.6, 0.16 mM NADH, 1.25 U glycerophosphate dehydrogenase and 12.5 U triose-1-phosphate isomerase. The absorption was monitored at 340 nm at 20° C. The molar adsorption coefficient taken was 6.22 mM⁻¹ cm⁻¹.

### HPLC

For time course studies, 20 µl of the reaction mixture were diluted 10 times before injection into the HPLC. The amount of phosphorylated products were determined by HPLC using an Alltech OA 1000 organic acid column (0.65 x 30 cm) equipped with a Dionex 580 LPG pump and Dionex UVD-340/Shodex RI-101 detector. The column was eluted with 25 mM H₂SO₄ at a flow rate of 0.4 mL min⁻¹. The HPLC effluent was monitored at 210, 215, 275 and 320 nm. The Chromeleon software program (Dionex) was used for HPLC data acquisition and evaluation.

### ³¹P-NMR

PPi, free phosphate, DHAP and phosphorylated aldol adduct were quantified by phosphor nuclear magnetic resonance (³¹P NMR). Spectra were determined on a Varian Unity Inova at 202 MHz using a 10 mm ¹⁵N-³¹P probe. Chemical shifts (δ) are expressed in ppm relative to 85% phosphoric acid. At zero time a spectrum was taken from the reaction mixture containing 240 mM PPi, 500 Mm DHA, 100 mM propionaldehyde and 100 mM sodium acetate buffer (pH 4.5) in a 10 mm NMR tube at 30° C. The reaction was initiated by addition of 2 µM PhoN-Sf. After 30 minutes, 10 units of RAMA were added. Concentrations of product and reactants were determined using 50 mM dimethylmethylphosphonate (DMMP) in deuterated water as an external standard, which was coaxially inserted in the NMR tube.

### Isolation and characterization of aldol product

Aldol product was formed as described above. The final mixture was extracted with ethyl acetate and purified by HPLC using a prevail carbohydrate ES 5u column (250 mm x 4.6 mm) equipped with a Dionex 50 LPG pump and dionex UVD-340/Shodex RI-101 detector. The column was eluted with 75% acetonitrile and a flow rate of 0.5 mL min⁻¹. The HPLC effluent was monitored at 210, 215, 275 and 320 nm. The Chromeleon software program (Dionex) was used for HPLC data acquisition and evaluation. The isolated product was characterized by ¹Hand ¹³C-NMR using a Bruker ARX 400 and by mass spectrometry carried out on a JEOL JMS-SX/SX 102 A Tandem Mass Spectrometer using Fast Atom Bombardment (FAB+) and identified as 5,6-dideoxy-D-threo-2-hexulose.

As an example of the versatility of our method we were able to synthesize a biologically active sugar analogue (deoxymannojirimycin) on an analytical scale in a two step chemo-enzymatic process using the phosphatase and the aldolase in a one pot procedure.

The reaction was started by the phosphatase-catalyzed phosphorylation of DHA by pyrophosphate to form DHAP and subsequent aldolase catalyzed condensation of 2-hydroxy-3-azido propionaldehyde to form a phosphorylated azido-aldol adduct. Upon prolonged incubation this adduct is then dephosphorylated by the phosphatase. After reduction with Pd in the presence of hydrogen the pure imino sugar 1-L-deoxymannojirimycin was formed. The identity of this compound was established by ¹³C-NMR and ¹H-NMR. The procedure that is outlined below in some detail may also be used to produce deoxynojirimycin and derivatives thereof. The derivatives of these imino sugar compounds are of particular interest since they are inhibitors of glycosidases and these inhibitors have many potential applications as antidiabetic, antiviral (HIV, influenza), and anticancer drugs.

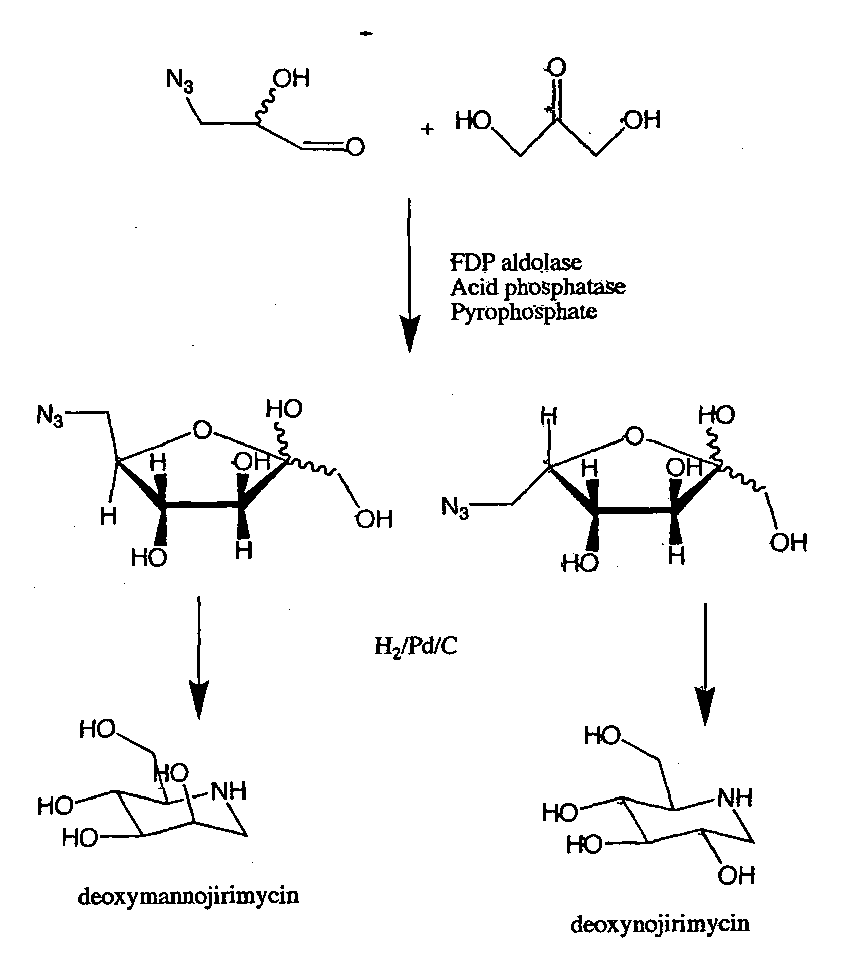

## Claims

1. A method for making dihydroxyacetone phosphate (DHAP) comprising treating dihydroxyacetone with a bacterial acid phosphatase in the presence of pyrophosphate.

2. The method according to claim 1 wherein the acid phosphatase is obtained from a bacterial species comprising the conserved sequence motifs domain 1 KXXXXXXRP, domain 2 PSGH and domain 3 SRXXXXXHXXXD.

3. The method according to claim 2 wherein the acid phosphatase is obtained from *Shigella flexneri* (PhoN-Sf).

4. The method according to any one of claims 1 to 3 wherein a suitable acidic pH is selected, after which during reacting the acid phosphatase and the dihydroxyacetone no further acid or base is added to change the pH.

5. A method for making stereospecifically a phosphate of the formula:
R-C*H (OH) -C*H (OH) -CO-CH₂-OPO₃H₂
comprising the steps:
a) treating dihydroxyacetone with a bacterial acid phosphatase in the presence of pyrophosphate to make dihydroxyacetone phosphate; and
b) treating the dihydroxyacetone phosphate with R-CHO and an aldolase, wherein R-CHO is any aldehydes, preferably R is selected from H, unsubstituted or substituted (cyclo) alkyl, and a carbohydrate moiety, and C* stands for a chiral carbon atom.

6. A method for making stereospecifically a compound of the formula:
R-C*H(OH)-C*H(OH)-CO-CH₂-OH
comprising the steps:
a) treating dihydroxyacetone with a bacterial acid phosphatase in the presence of pyrophosphate to make dihydroxyacetone phosphate;
b) treating the dihydroxyacetone phosphate with R-CHO and an aldolase, wherein R-CHO is any aldehydes, preferably R is selected from H, unsubstituted or substituted (cyclo) alkyl, and a carbohydrate moiety, and C* stands for a chiral carbon atom to a phosphate of the formula: R-C*H(OH)-C*H(OH)-CO-CH₂-OPO₃H₂; and
c) dephosphorylating the phosphate of step b) by treading said phosphate with a bacterial acid phosphatase.

7. The method according to claim 5 or 6 as performed in a one-pot procedure.

8. The method according to claim 6 or 7 for making carbohydrates by treating the dihydroxyacetone phosphate in step b) with an aldehyde and an aldolase, and treating the phosphate in step c) with acid phosphatase.

9. The method according to claim 8 wherein the acid phosphatase of step c) is obtained from a bacterial species comprising the conserved sequence motifs domain 1 KXXXXXXRP, domain 2 PSGH and domain 3 SRXXXXXHXXXD, preferably from *Shigella flexneri* (PhoN-Sf) .

10. The method according to claim 8 or 9 wherein the aldolase is a DHAP-dependent aldolase belonging to the class selected from D-fructose 1,6-bis-phosphate aldolase (RAMA), rhamnulose 1-phosphate aldolase, D-tagatose 1,6-bis-phosphate aldolase, and L-fuculose 1-phosphate aldolase.

## Patentansprüche

1. Ein Verfahren zur Herstellung von Dihydroxyacetonphosphat (DHAP), das das Behandeln von Dihydroxyaceton mit einer bakteriellen sauren Phosphatase in Gegenwart von Pyrophosphat umfasst.

2. Das Verfahren gemäß Anspruch 1, wobei die saure Phosphatase von einer bakteriellen Spezies erhalten wird, die die konservierten Sequenzmotive Domäne 1 KXXXXXXRP, Domäne 2 PSGH und Domäne 3 SRXXXXXHXXXD umfasst.

3. Das Verfahren gemäß Anspruch 2, wobei die saure Phosphatase von *Shigella flexneri* (PhoN-Sf) erhalten wird.

4. Das Verfahren gemäß einem der Ansprüche 1 bis 3, wobei ein geeigneter saurer pH ausgewählt wird, wonach während der Umsetzung der sauren Phosphatase und des Dihydroxyacetons keine weitere Säure oder Base hinzugegeben wird, um den pH zu ändern.

5. Ein Verfahren zur stereospezifischen Herstellung eines Phosphats der Formel:
R-C*H(OH)-C*H(OH)-CO-CH₂-OPO₃H₂
dass die Schritte umfasst:
a) Behandeln von Dihydroxyaceton mit einer bakteriellen sauren Phosphatase in Gegenwart von Pyrophosphat, um Dihydroxyacetonphosphat herzustellen; und
b) Behandeln des Dihydroxyacetonphosphats mit R-CHO und einer Aldolase, wobei R-CHO ein Aldehyd ist, wobei bevorzugt R aus H, unsubstituiertem oder substituiertem (Cyclo)alkyl und einer Kohlenhydrateinheit ausgewählt wird und C* für ein chirales Kohlenstoffatom steht.

6. Ein Verfahren zur stereospezifischen Herstellung einer Verbindung der Formel:
R-C*H(OH)-C*H(OH)-CO-CH₂-OH
dass die Schritte umfasst:
a) Behandeln von Dihydroxyaceton mit einer bakteriellen sauren Phosphatase in Gegenwart eines Pyrophosphats, um Dihydroxyacetonphosphat herzustellen; und
b) Behandeln des Dihydroxyacetonphosphats mit R-CHO und einer Aldolase, wobei R-CHO ein Aldehyd ist, wobei bevorzugt R aus H, unsubstituiertem oder substituiertem (Cyclo)alkyl und einer Kohlenhydrateinheit ausgewählt wird und C* für ein chirales Kohlenstoffatom steht, zu einem Phosphat der Formel R-C*H(OH)-C*H(OH)-CO-CH₂-OPO₃H₂; und
c) Dephosphorylieren des Phosphats von Schritt b) durch Behandeln des Phosphats mit einer bakteriellen sauren Phosphatase.

7. Das Verfahren gemäß Anspruch 5 oder 6 wie in einem Ein-Topf-Verfahren durchgeführt.

8. Das Verfahren gemäß Anspruch 6 oder 7 zur Herstellung von Kohlenhydraten durch Behandeln des Dihydroxyacetonphosphats in Schritt b) mit einem Aldehyd und einer Aldolase und Behandeln des Phosphats in Schritt c) mit saurer Phosphatase.

9. Das Verfahren gemäß Anspruch 8, wobei die saure Phosphatase von Schritt c) von einer bakteriellen Spezies erhalten wird, die die konservierten Sequenzmotive Domäne 1 KXXXXXXRP, Domäne 2 PSGH und Domäne 3 SRXXXXXHXXXD umfasst, bevorzugt von *Shigella flexneri* (PhoN-Sf).

10. Das Verfahren gemäß Anspruch 8 oder 9, wobei die Aldolase eine DHAP-abhängige Aldolase ist, die zur der Klasse gehört, die aus D-Fruktose-1,6-bisphosphataldolase (RAMA), Rhamnulose-1-phosphataldolase, D-Tagatose-1,6-bis-phosphataldolase und L-Fuculose-1-phosphataldolase ausgewählt wird.

## Revendications

1. Procédé de préparation de dihydroxyacétone phosphate (DHAP) comprenant le traitement de la dihydroxyacétone avec une phosphatase acide bactérienne en présence de pyrophosphate.

2. Procédé selon la revendication 1, dans lequel la phosphatase acide est obtenue à partir d'une espèce bactérienne comprenant les motifs de séquence conservés domaine 1 KXXXXXXRP, domaine 2 PSGH et domaine 3 SRXXXXXHXXXD.

3. Procédé selon la revendication 2, dans lequel la phosphatase acide est obtenue à partir de *Shigella flexneri* (PhoN-Sf).

4. Procédé selon l'une quelconque des revendications 1 à 3, dans lequel un pH acide approprié est sélectionné, après quoi, au cours de la réaction de la phosphatase acide et de la dihydroxyacétone, aucun autre acide ou aucune autre base n'est ajoutée pour modifier le pH.

5. Procédé de préparation stéréospécifique d'un phosphate de formule :
R-C*H(OH)-C*H(OH)-CO-CH₂-OPO₃H₂
comprenant les étapes consistant à :
a) traiter la dihydroxyacétone avec une phosphatase acide bactérienne en présence de pyrophosphate pour préparer du dihydroxyacétone phosphate ; et
b) traiter le dihydroxyacétone phosphate avec R-CHO et une aldolase, où R-CHO est un aldéhyde quelconque, de préférence R est choisi parmi H, (cyclo)alkyle non substitué ou substitué, et une fraction hydrate de carbone, et -C* représente un atome de carbone chiral.

6. Procédé de préparation stéréospécifique d'un composé de formule :
R-C*H(OH)-C*H(OH)-CO-CH₂-OH
comprenant les étapes consistant à :
a) traiter la dihydroxyacétone avec une phosphatase acide bactérienne en présence de pyrophosphate pour préparer du dihydroxyacétone phosphate ;
b) traiter le dihydroxyacétone phosphate avec R-CHO et une aldolase, où R-CHO est un aldéhyde quelconque, de préférence R est choisi parmi H, (cyclo)alkyle non substitué ou substitué, et une fraction hydrate de carbone, et C* représente un atome de carbone chiral à un phosphate de formule :
R-C*H(OH)-C*H(OH)-CO-CH₂-OPO₃H₂ ; et
c) déphosphoryler le phosphate de l'étape b) en traitant ledit phosphate avec une phosphatase acide bactérienne.

7. Procédé selon la revendication 5 ou 6 tel qu'il est réalisé dans une procédure en un pot.

8. Procédé selon la revendication 6 ou 7 pour la préparation d'hydrates de carbone en traitant le dihydroxyacétone phosphate à l'étape b) avec un aldéhyde ou une aldolase, et en traitement le phosphate à l'étape c) avec une phosphatase acide.

9. Procédé selon la revendication 8, dans lequel la phosphatase acide de l'étape c) est obtenue à partir d'une espèce bactérienne comprenant les motifs de séquence conservés domaine 1 KXXXXXXRP, domaine 2 PSGH et domaine 3 SRXXXXXHXXXD, de préférence de *Shigella flexneri* (PhoN-Sf).

10. Procédé selon la revendication 8 ou 9, dans lequel l'aldolase est une aldolase DHAP-dépendante appartenant à la classe choisie parmi la D-fructose 1,6-bis-phosphate aldolase (RAMA), la rhamnulose 1-phosphate aldolase, la D-tagatose 1,6-bis-phosphate aldolase, et la L-fuculose 1-phosphate aldolase.
